Europäisches Patentamt

European Patent Office

Office européen des brevets

(19)

(11) Veröffentlichungsnummer: **0 216 180 B1**

(12)

# EUROPÄISCHE PATENTSCHRIFT

(45) Veröffentlichungstag der Patentschrift: **11.03.92**

(51) Int. Cl.⁵: **C07C 39/12**, C07C 37/07

(21) Anmeldenummer: **86111853.7**

(22) Anmeldetag: **27.08.86**

(54) **Neue Herstellung von Alkyltetrahydro-Anthrahydrochinonen.**

(30) Priorität: **24.09.85 DE 3534014**

(43) Veröffentlichungstag der Anmeldung:
**01.04.87 Patentblatt 87/14**

(45) Bekanntmachung des Hinweises auf die
Patenterteilung:
**11.03.92 Patentblatt 92/11**

(84) Benannte Vertragsstaaten:
**AT BE CH DE FR GB IT LI NL SE**

(56) Entgegenhaltungen:
DE-A- 2 426 879
FR-A- 2 112 281

**CHEMICAL ABSTRACTS, Band 95, 1981, Seite
617, Nr. 203235q, Columbus, Ohio, US; Y. LI
et al.: "Poly(vinylpyrrolidone)-palladium(II)
complex as homogeneous and heterogeneous hydrogenation catalysts for alkenes"**

(73) Patentinhaber: **Peroxid-Chemie GmbH
Dr.-Gustav-Adolph-Strasse 2
W-8023 Höllriegelskreuth bei München(DE)**

(72) Erfinder: **Simon, Dietolf
Am Kleinen Bäumchen 7
W-5462 Bad Hönningen(DE)**

(74) Vertreter: **Weickmann, Heinrich, Dipl.-Ing. et
al
Patentanwälte H.Weickmann, Dr. K.Fincke
F.A. Weickmann, B. Huber Dr. H. Liska, Dr. J.
Prechtel Kopernikusstrasse 9 Postfach 86 08
20
W-8000 München 86(DE)**

## Beschreibung

Die Erfindung betrifft ein Verfahren zur Herstellung von Alkyl-5,6,7,8-tetrahydroanthrahydrochinonen.

Es ist bekannt, Alkylanthrachinone in Mischung mit den hydrierstabileren Alkyltetrahydroanthrachinonen zur Herstellung von $H_2O_2$ nach dem Anthrachinonverfahren zu verwenden. Die Tetrahydroanthrachinone entstehen dabei sukzessive von selbst im Verfahrenscyclus als Hydriernebenprodukt der Alkylanthrachinone. Jedoch war es bisher nicht möglich, mit einem einfachen Verfahren Alkyltetrahydroanthrahydrochinone herzustellen. Es war daher wünschenswert, die Tetrahydroderivate in einer separaten Synthese gezielt unter optimierten Hydrierbedingungen herzustellen und direkt im Anthrachinonverfahren einzusetzen. Ziel der Erfindung war es nun, ein Verfahren zu schaffen, mit dem Alkylanthrachinone in hoher Ausbeute zu den Tetrahydroderivaten hydriert werden können und bei dem andererseits die bekannten Nachteile der üblicherweise heterokatalytisch geführten Hydrierung, z.B. durchgeführt gemäß FR-A 2 112 281, wie Adsorptions/Desorptionsprobleme, Uneinheitlichkeit der Produktbildung durch Inhomogenität der katalytischen Reaktionszentren, vermieden werden.

Dieses Ziel wird erreicht durch ein Verfahren zur Herstellung von Alkyl-5,6,7,8-tetrahydroanthrahydrochinonen, das dadurch gekennzeichnet ist, daß man Alkylanthrachinone mit einem in wäßriger oder organischer Lösung vorliegenden Katalysator, der aus einem organischen Polymer besteht, an das ein Metall der Edelmetall- oder Platinmetallgruppe koordinativ oder kovalent gebunden ist, hydriert.

Dieses Verfahren ist sehr einfach durchzuführen und liefert in hochselektiver Weise die Tetrahydroderivate in der Hydrochinonform. Anschließende Oxidation der Tetrahydroanthrahydrochinone mit einem sauerstoffhaltigen Gas ergibt in bekannter Weise die entsprechenden Tetrahydroanthrachinone. Besonders vorteilhaft ist es jedoch, die Tetrahydroanthrahydrochinone als solche direkt im Anthrachinonverfahren einzusetzen, in dessen Verfahrensablauf sie dann zu Tetrahydroanthrachinon und $H_2O_2$ umgesetzt werden.

Bei der Durchführung des erfindungsgemäßen Verfahrens wird das zu hydrierende Alkylanthrachinon entweder in einem alle Komponenten lösenden Lösungsmittel, insbesondere einem Alkohol mit 1 bis 10 C-Atomen, bevorzugt 1 bis 4 C-Atomen, bzw. einem cyclischen oder acyclischen Säureamid oder in einem mit Wasser nicht mischbaren Lösungsmittelsystem gelöst. In letzterem Fall wird der Katalysator in wässriger Lösung vorgelegt; die Hydrierung erfolgt dann im emulgierten Zweiphasensystem aus wässriger Katalysatorlösung und organischer Alkylanthrachinonlösung.

Beispiele für geeignete Lösungsmittel sind Alkohole, insbesondere mit 1 bis 10 Kohlenstoffatomen, wie Methanol, Äthanol, Propanol, Isopropanol, Butanol, t-Butanol, Isobutanol und Diisobutylcarbinol, hydrierstabile Ketone wie Diisobutylketon, Cyclohexanon, aromatische, alicyclische und/oder aliphatische Kohlenwasserstoffe und deren Alkoxy- bzw. Halogenderivate wie insbesondere alkylierte oder alkoxylierte Benzole oder Naphtaline und/oder cyclische oder acyclische Säureamide oder tetrasubstituierte Harnstoffe wie Tetramethylharnstoff usw. Besonders bevorzugt werden Alkohole mit 1 bis 4 Kohlenstoffatomen bzw. Säureamide wie N-Methylpyrrolidon und Dimethylformamid für die homogene Mischung und Gemische von Diisobutylcarbinol mit Alkyl-Aromaten für die heterogene Arbeitsweise mit Emulsionsbildung (wäßrige Katalysatorlösung) verwendet.

Die Hydrierung erfolgt mit einem in wäßriger oder organischer Lösung vorliegenden Katalysator. Der Katalysator besteht aus einem organischen Polymer, an das ein Metall der Edelmetall- oder Platinmetallgruppe koordinativ gebunden ist. Ein derartiger Katalysator kann beispielsweise hergestellt werden, indem die wäßrige Lösung des organischen Polymers mit dem entsprechenden Metallsalz gemischt wird und dann durch Wasserstoffeinleitung das Metall molekular auf dem Träger ausgefällt wird. Als Metallsalz wird beispielsweise das entsprechende Chlorid eingesetzt.

Als Trägermaterialien werden organische Polymere verwendet. Bevorzugt werden als Polymere Polyvinylpyrrolidon, Polyethylenimin, Carboxymethylcellulose, Polyvinylalkohol, Polyvinylamin und/oder Amylose verwendet. Diese Polymere können auch in substituierter Form vorliegen. So kann beispielsweise Polyethylenimin am Stickstoff durch Umsetzung mit Chloressigsäure substituiert sein. Besonders bevorzugt wird Polyvinylpyrrolidon. Besonders bevorzugt ist es weiter, als organischen polymeren Träger für den Katalysator Polymere mit einem Molekulargewicht von mehr als 100.000, bevorzugt mehr als 300.000 zu verwenden. Diese hochmolekularen Polymere lassen sich dann leicht nach Abschluß der Hydrierungsreaktion durch Ultrafiltration abtrennen.

Die katalytisch aktiven Metalle, die an das Polymer koordinativ gebunden sind, entstammen der Edelmetall- oder Platinmetallgruppe. Bevorzugt werden als katalytisch aktive Metalle Rhodium, Ruthenium, Palladium, Osmium, Iridium und/oder Platin verwendet, mehr bevorzugt Rhodium oder/und Palladium.

Besonders bevorzugt wird als Katalysator Rhodium, das an Polyvinylpyrrolidon gebunden ist, verwendet.

Die Hydrierung der Alkylanthrachinone erfolgt entweder in dem aus dem wasserunmischbaren Lösungsmittelsystem und der wäßrigen Lösung gebildeten Zweiphasensystem (Emulsionshydrierung) oder im homogenen Einphasensystem. Das heterogene System hat den Vorteil, leicht aufarbeitbar zu sein durch Trennung der Phasen, im homogenen System dagegen werden bessere Katalysatorstandzeiten erreicht.

Die Konzentration des Katalysators liegt bevorzugt in einem Bereich von $10^{-2}$ bis $10^{-4}$ g Atom Metall pro l Hydrierlösung. Besonders bevorzugt wird das an das Polymer gebundene Metall in einer Menge von $10^{-3}$ bis $10^{-4}$ g Atom Metall pro l Hydrierlösung verwendet.

Bei dem erfindungsgemäßen Verfahren zur Hydrierung wird das organische Polymer bevorzugt mit einer Menge von 0,5 bis 10 Gew.-% bezogen auf das Polymer mit Metall beladen. Besonders bevorzugt beträgt die Metallbeladung l bis 6 Gew.-% Metall, bezogen auf das Polymer.

Als Alkylanthrachinone, die hydriert werden, werden bevorzugt die in Zweistellung alkylierten Anthrachinone verwendet. Besonders bevorzugt werden Anthrachinone hydriert, die in Zweistellung mit einer $C_1$- bis $C_{10}$-Alkylgruppe, insbesondere mit einer Ethyl-, t-Butyl- oder Amylgruppe substituiert sind.

Das erfindungsgemäße Verfahren kann ohne Druckerhöhung und bei Raumtemperatur durchgeführt werden. Bevorzugt wird die Hydrierung jedoch bei einem Druck von 0,5 bis 5 MPa, besonders bevorzugt 1 bis 4 MPa und bei einer Temperatur von 20 bis 70°C durchgeführt.

Besonders bevorzugt wird die Hydrierung mit einem Wasserstoffdruck von 1,5 bis 2,5 MPa durchgeführt. Bei diesem Druck ist das Verhältnis von Ausbeute, Druckhöhe und Geschwindigkeit der Umsetzung am günstigsten. Die Temperatur der Hydrierstufe liegt besonders bevorzugt im Bereich zwischen 40 und 60°C.

In einer bevorzugten Ausführungsform des erfindungsgemäßen Verfahrens, bei der als Hydrierkontakt ein Katalysator auf PVP-Basis verwendet wird, wird die Geschwindigkeit der Hydrierreaktion noch durch katalytische Säuremengen beeinflußt. Die Zugabe einer geringen Säuremenge führt zu einer Steigerung der Umsatzgeschwindigkeit. Ausreichend ist beispielsweise der Zusatz von 0,1 ml Schwefelsäure pro Liter Lösung.

Wenn die Hydrierungsrekation abgeschlossen ist, wird das hydrierte Produkt, das Alkyl-tetrahydroanthrahydrochinon, aus dem Reaktionsgemisch abgetrennt. Im Falle der Emulsionshydrierung wird zunächst die wässrige Katalysatorlösung separiert, was durch einfache Dekantation und gegebenenfalls anschließende Zentrifugation erfolgen kann. Die Abtrennung des Tetrahydroanthrahydrochinons aus dem organischen Lösungsmittel kann wahlweise durch Kristallisation in der Kälte oder durch Einengen der Lösung bzw. Zusatz eines Fällungsmittels geschehen.

Wird im homogenen Einphasensystem hydriert, so wird der Katalysator zweckmäßig durch Ultrafiltration oder Extraktion abgetrennt und dann das Produkt durch Einengen der filtrierten bzw. extrahierten Lösung, zweckmäßig im Vakuum, und Kristallisation aus dem Konzentrat gewonnen.

Zur Durchführung des erfindungsgemäßen Verfahrens wird der Katalysator im gewählten organischen oder wäßrigen Lösungsmittel gelöst und diese Lösung dann mehrere Stunden mit Wasserstoff bei Normaldruck und Raumtemperatur behandelt, wobei sich eine Dunkelfärbung ergibt. Der so aktivierten Katalysatorlösung wird bei Arbeiten in homogener Phase dann das Alkylanthrachinon zugegeben und unter Erwärmen gelöst. Wenn ein Katalysator auf Polyvinylpyrrolidonbasis verwendet wird, wird vorzugsweise eine katalytische Säuremenge zugegeben und das erhaltene Reaktionsgemisch im Rührautoklaven hydriert. Bei Arbeiten in heterogener Phase wird nach der Aktivierung der mit wässriger Katalysatorlösung mit Wasserstoff die Lösung des Alkylanthrachinons im organischen Lösungsmittelsystem zugesetzt und die weitere Hydrierung, gegebenenfalls nach Zusatz katalytischer Säuremengen, unter intensivem Rühren im Autoklaven durchgeführt. Im Falle der Emulsionshydrierung trennen sich nach Beendigung des Rührens die Phasen und können leicht abdekantiert werden. Zweckmäßig wird die abgetrennte organische Phase noch zentrifugiert, um emulgierte Katalysator-Lösungsreste möglichst quantitativ zu entfernen.

Die Katalysatorherstellung selbst kann beispielsweise erfolgen, indem man das gewählte Polymer, beispielsweise Polyvinylpyrrolidon (PVP) in wäßriger oder alkoholischer Lösung mit einem entsprechenden Edelmetallsalz, beispielsweise dem Chlorid des jeweiligen Edelmetalls mischt und dann durch Wasserstoffeinleitung das Metall molekular auf dem Träger ausfällt.

Das erfindungsgemäße Verfahren zeichnet sich durch hohe Ausbeuten (praktisch quantitativ) aus. Es sind sehr viele Hydrierzyklen möglich, ohne daß die Katalysatoraktivität deutlich nachläßt. Durch die hohe Selektivität, die fast 100 % erreicht, wird die Bildung von Nebenprodukten, die das Endprodukt verunreinigen könnten, minimal gehalten.

Die Erfindung wird anhand der folgenden Beispiele näher erläutert.

**Beispiel 1**

2-Alkylanthrachinon, dessen jeweilige Zusammensetzung der Tabelle 1 zu entnehmen ist, wurde in homogener Phase mit Katalysatoren auf Polyvinylpyrrolidonbasis hydriert. Die Hydrierungen erfolgten nach Zusatz von 0,4 ml $H_2SO_4$ Konz./l Lösung unter Variation von Katalysator Metall, Metallgehalt, Chinon, Chinonkonzentration, Temperatur, Druck, Lösungsmittel und Reaktionsdauer. Tabelle 1 gibt einen Uberblick über die erhaltenen Ergebnisse.

T a b e l l e  1

| Versuch Nr. | Katalysatormetall* Typ | Gehalt g Atom Metall/l | Alkylanthrachinon Alkylrest | Gehalt mol/l | Temperatur °C | Druck MPa | Lösungsmittel | Dauer h | Hydrierumsatz** % |
|---|---|---|---|---|---|---|---|---|---|
| 1 | Rh | $29.2 \times 10^{-4}$ | Amyl | $6.8 \times 10^{-2}$ | 50 | 1 | Ethanol | 2 | 65 |
| 2 | Rh | " | Amyl | " | " | " | " | 4 | 94 |
| 3 | " | " | " | " | " | 2,5 | " | " | 100 |
| 4 | " | " | " | " | " | 1 | Butanol | 2 | 51 |
| 5 | " | " | " | " | " | " | " | 4 | 92 |
| 6 | Pd | " | " | " | " | 0,5 | Ethanol | " | 65 |
| 7 | " | " | " | " | " | 1 | " | " | 94 |
| 8 | " | " | " | " | " | 2,5 | " | " | 100 |
| 9 | Rh | $6.5 \times 10^{-4}$ | Ethyl | $28,0 \times 10^{-2}$ | 40 | 2,0 | N-Methyl-pyrrolidon | " | 31 |
| 10 | " | $13.0 \times 10^{-4}$ | " | " | 60 | " | " | " | 66 |
| 11 | " | $6.5 \times 10^{-4}$ | " | " | 40 | " | Dimethylform-amid | " | 25 |
| 12 | " | $13.0 \times 10^{-4}$ | " | " | 60 | " | " | " | 49 |

*) Metallbeladung des Polymers: 6 Gew.%  Versuche 1 – 8
   1 Gew.%  Versuche 9 – 12

**) bez. auf die eingesetzte Menge an Alkylanthrachinon

**Beispiel 2**

Die Selektivität des erfindungsgemäßen Hydrierverfahrens wurde am Beispiel der homogenen Umsetzung mit Rhodium-Polyvinylpyrrolidon in Butanol als Lösungsmittel untersucht. Hydriert wurde mit einem Katalysator-Metallgehalt von 29,2 x $10^{-4}$ g Atom Metall/l Hydrierlösung, wobei die Metallbeladung des Polymers in allen Fällen 6 Gew.-% betrug. Der Alkylanthrachinongehalt betrug 60,5 x $10^{-3}$ mol/l Lösung; die Hydrierung erfolgte nach Zusatz von 0,4 ml $H_2SO_4$ Konz. zu 1 l Hydriergemisch. Die nachfolgende Tabelle 2 gibt die bei praktisch vollständigem Hydrierumsatz des Alkylanthrachinons unter verschiedenen Reaktionsbedingungen erhaltenen Ausbeuten an Tetrahydroderivat an.

Tabelle 2

| Versuch Nr. | Alkylrest/Anthrachinon | Druck MPa | Temperatur °C | Dauer h | Ausbeute an Tetrahydroderivat/% |
|---|---|---|---|---|---|
| 1 | t-Butyl | 1 | 50 | 4 | 91,0 |
| 2 | " | " | " | 6 | 98,5 |
| 3 | " | " | " | 8 | 98,0 |
| 4 | " | 2,1 | 60 | 1,5 | 98,0 |
| 5 | " | " | " | 3,5 | 98,7 |
| 6 | Ethyl | 2,5 | 50 | 2 | 97,4 |
| 7 | " | " | " | 4 | 98,2 |

Es zeigte sich, daß dieses Hydriersystem sehr stabil ist. Nach 6 Hydriercyclen in Folge mit derselben Katalysatorprobe zeigte sich keinerlei Aktivitätsverlust des Katalysators.

**Beispiel 3**

Hydrierung in heterogener Phase

Unter Verwendung von Rh/PVP in 150 ml $H_2O$ gelöst und 2-Amylanthrachinon(24 x $10^{-2}$ mol/l) gelöst in dem in der nachstehenden Tabelle 3 angegebenen organischen Lösungsmittel, wurde in emulgierter Phase eine Hydrierung bei 50°C über 4 Stunden durchgeführt. Als Aktivator wurden 0,4 ml $H_2SO_4$ Konz. zugesetzt. Nachstehende Tabelle 3 gibt die unter verschiedenen Reaktionsbedingungen erhaltenen Hydrierresultate wieder.

Tabelle 3

| Versuch | % Metallbeladung/Polymer | Rh-Gehalt* g Atom/l | Lösungsmittel für Alkylanthrachinon | Druck MPa | Hydrierumsatz % |
|---|---|---|---|---|---|
| 1 | 6 | $4,9 \times 10^{-3}$ | Diisobutylcarbinol/C 10-Aromatengemisch | 4,0 | 100 |
| 2 | 1 | " | " | 2,5 | 85 |
| 3 | 1 | $1,9 \times 10^{-3}$ | Cyclohexanon | 2,5 | 68 |
| 4 | 1 | " | Tetralin | " | 19 |
| 5 | 1 | " | $CCl_2F - CCl_2F$ | " | 13 |

*) Gehalt pro 1 wässrige Katalysatorlösung

**Patentansprüche**

1. Verfahren zur Herstellung von Alkyl-5,6,7,8-tetrahydroanthrahydrochinonen, **dadurch gekennzeichnet,** daß man Alkylanthrachinone mit einem in wäßriger oder organischer Lösung vorliegenden Katalysator, der aus einem organischen Polymer besteht, an das ein Metall der Edelmetall- oder Platinmetallgruppe koordinativ oder kovalent gebunden ist, hydriert.

2. Verfahren nach Anspruch 1, **dadurch gekennzeichnet,** daß man 2-Alkyl-Anthrachinon verwendet.

3. Verfahren nach Anspruch 1, **dadurch gekennzeichnet,** daß man als Lösungsmittel für die Anthrachinone Alkohole, hydrierstabile Ketone, aromatische, alicyclische und/oder aliphatische Kohlenwasserstoffe und deren Alkoxy- bzw. Halogenderivate, cyclische oder acyclische Säureamide und/oder tetrasubstituierte Harnstoffverbindungen verwendet.

4. Verfahren nach Anspruch 3, **dadurch gekennzeichnet,** daß man in homogener Phase mit Alkoholen mit 1 bis 10 C-Atomen und/oder Säureamiden als Lösungsmittel hydriert.

5. Verfahren nach Anspruch 3, **dadurch gekennzeichnet,** daß man in heterogener Phase mit einer Emulsion aus wäßriger Katalysatorlösung und einer Alkylanthrachinonlösung in einem organischen, mit Wasser nicht mischbaren Lösungsmittel oder -gemisch hydriert.

6. Verfahren nach einem der vorhergehenden Ansprüche, **dadurch gekennzeichnet,** daß man als organisches Polymer Polyvinylpyrrolidon, Polyethylenimin, Carboxymethylcellulose, Polyvinylalkohol, Polyvinylamin und/oder Amylose und/oder ein Derivat dieser Polymeren verwendet.

7. Verfahren nach einem der vorhergehenden Ansprüche, **dadurch gekennzeichnet,** daß das Metall Ruthenium, Rhodium, Palladium, Osmium, Iridium und/oder Platin ist.

8. Verfahren nach Anspruch 6 oder 7, **dadurch gekennzeichnet,** daß man Rhodium oder Palladium gebunden an Polyvinylpyrrolidon verwendet.

9. Verfahren nach einem der vorhergehenden Ansprüche, **dadurch gekennzeichnet,** daß man mit einer Konzentration an Polymer -gebundenem Metall von $10^{-2}$ bis $10^{-4}$ g Atom Metall pro l Hydrierlösung hydriert.

10. Verfahren nach einem der vorhergehenden Ansprüche, **dadurch gekennzeichnet,** daß die Metallbeladung 0,5 bis 10 Gew.-%, bezogen auf das Polymer, beträgt.

11. Verfahren nach einem der vorhergehenden Ansprüche, **dadurch gekennzeichnet,** daß man die Hydrierung mit einem Wasserstoffdruck von 0,5 - 5 MPa durchführt.

12. Verfahren nach einem der vorhergehenden Ansprüche, **dadurch gekennzeichnet**, daß man die Hydrierung bei einer Temperatur von 20 bis 70 °C durchführt.

13. Verfahren nach einem der vorhergehenden Ansprüche, **dadurch gekennzeichnet,** daß man mit einem Katalysator auf Polyvinylpyrrolidon-Basis hydriert und zusätzlich katalytische Säuremengen zusetzt.

14. Verfahren nach einem der vorhergehenden Ansprüche, **dadurch gekennzeichnet,** daß das Alkyl-5,6,7,8-tetrahydro-anthrahydrochinon durch Kristallisation, Ultrafiltration, Phasendekantation oder Extraktion abgetrennt wird.

**Claims**

1. Process for the preparation of alkyl-5,6,7,8-tetrahydroanthraquinones, characterised in that one hydrogenates alkylanthraquinones with a catalyst, present in aqueous or organic solution, which consists of an organic polymer to which a metal of the noble metal or platinum group is bound coordinatively or covalently.

2. Process according to claim 1, characterised in that one uses 2-alkylanthraquinone.

3. Process according to claim 1, characterised in that, as solvent for the anthraquinone, one uses alcohols, hydrogenation-stable ketones, aromatic, alicyclic and/or aliphatic hydrocarbons or their alkyl or halogen derivative, cyclic or acyclic acid amides and/or a tetrasubstituted urea compound.

4. Process according to claim 3, characterised in that one hydrogenates in homogeneous phase with alcohols with 1 to 10 C-atoms and/or acid amides as solvent.

5. Process according to claim 3, characterised in that one hydrogenates in heterogeneous phase with an emulsion of aqueous catalyst solution and of an alkylanthraquinone solution in an organic solvent or solvent mixture not miscible with water.

6. Process according to one of the preceding claims, characterised in that, as organic polymer, one uses polyvinylpyrrolidone, polyethyleneimine, carboxymethylcellulose, polyvinyl alcohol, polyvinylamine and/or amylose and/or a derivative of these polymers.

7. Process according to one of the preceding claims, characterised in that the metal is ruthenium, rhodium, palladium, osmium, iridium and/or platinum.

8. Process according to claim 6 or 7, characterised in that one uses rhodium or palladium bound to polyvinylpyrrolidone.

9. Process according to one of the preceding claims, characterised in that one hydrogenates with a concentration of polymer-bound metal of from $10^{-2}$ to $10^{-4}$ g atom of metal per l of hydrogenation solution.

10. Process according to one of the preceding claims, characterised in that the metal loading amounts to 0.5 to 10 wt.%, referred to the polymer.

11. Process according to one of the preceding claims, characterised in that one carries out the hydrogenation with a hydrogen pressure of 0.5 - 5 MPa.

12. Process according to one of the preceding claims, characterised in that one carries out the hydrogenation at a temperature of 20 to 70°C.

13. Process according to one of the preceding claims, characterised in that one hydrogenates with a catalyst based on polyvinylpyrrolidone and additionally adds catalytic amounts of acid.

14. Process according to one of the preceding claims, characterised in that the alkyl-5,6,7,8-tetrahydroanthraquinone is separated by crystallisation, ultrafiltration, phase decantation or extraction.

## Revendications

1. Procédé de préparation d'alkyl-5,6,7,8-tétrahydroanthrahydroquinones, caractérisé en ce que l'on hydrogène des alkylanthraquinones avec un catalyseur qui consiste en un polymère organique, présent dans une solution aqueuse ou organique, auquel est lié par coordination ou covalence un métal du groupe des métaux nobles ou du platine.

2. Procédé selon la revendication 1, caractérisé en ce que l'on utilise une 2-alkylanthraquinone.

3. Procédé selon la revendication 1, caractérisé en ce que l'on utilise comme solvant pour les anthraquinones des alcools, des cétones stables à l'hydrogénation, des hydrocarbures aromatiques, alicycliques et/ou aliphatiques et leurs dérivés alcoxy ou halogénés, des amides d'acide cycliques ou acycliques et/ou des composés tétrasubstitués de l'urée.

**4.** Procédé selon la revendication 3, caractérisé en ce que l'on hydrogène en phase homogène avec des alcools ayant 1 à 10 atomes de carbone et/ou des amides d'acide en tant que solvant.

**5.** Procédé selon la revendication 3, caractérisé en ce que l'on hydrogène en phase hétérogène avec une émulsion d'une solution aqueuse de catalyseur et d'une solution d'alkylanthraquinone dans un solvant ou mélange de solvants organiques, immiscibles à l'eau.

**6.** Procédé selon l'une des revendications précédentes, caractérisé en ce que l'on utilise comme polymère organique la polyvinylpyrrolidone, la polyéthylèneimine, la carboxyméthylcellulose, l'alcool polyvinylique, la polyvinylamine et/ou l'amylose et/ou un dérivé de ces polymères.

**7.** Procédé selon l'une des revendications précédentes, caractérisé en ce que le métal est le ruthénium, le rhodium, le palladium, l'osmium, l'iridium et/ou le platine.

**8.** Procédé selon la revendication 6 ou 7, caractérisé en ce que l'on utilise le rhodium ou le palladium lié à la polyvinylpyrrolidone.

**9.** Procédé selon l'une des revendications précédentes, caractérisé en ce que l'on hydrogène avec une concentration en métal lié au polymère de $10^{-2}$ à $10^{-4}$ atome-gramme de métal par litre de solution d'hydrogénation.

**10.** Procédé selon l'une des revendications précédentes, caractérisé en ce que la charge métallique est de 0,5 à 10% en poids par rapport au polymère.

**11.** Procédé selon l'une des revendications précédentes, caractérisé en ce que l'on effectue l'hydrogénation avec une pression d'hydrogène de 0,5 à 5 MPa.

**12.** Procédé selon l'une des revendications précédentes, caractérisé en ce que l'on effectue l'hydrogénation à une température de 20 à 70°C.

**13.** Procédé selon l'une des revendications précédentes, caractérisé en ce que l'on hydrogène avec un catalyseur à base de polyvinylpyrrolidone et on ajoute en plus des quantités catalytiques d'acide.

**14.** Procédé selon l'une des revendications précédentes, caractérisé en ce que l'alkyl-5,6,7,8-tétrahydroanthrahydroquinone est séparée par cristallisation, ultrafiltration, décantation de phases ou extraction.